# EUROPÄISCHE PATENTANMELDUNG

(11) **EP 0 743 048 A2**
(43) Veröffentlichungstag der Anmeldung: **20.11.1996**
(21) Anmeldenummer: 96111669.6
(22) Anmeldetag: 18.12.1992
(51) Int. Cl.: A61F 2/30

(54) **Zentrierelement für Knochenimplantate**

(62) Teilanmeldung aus: 92121599.2
(71) Anmelder: Härle, Anton, Prof. Dr., D-48161 Münster (DE)
(72) Erfinder: Härle, Anton, Prof. Dr., D-48161 Münster (DE)

(57) **Zusammenfassung**

Die Erfindung betrifft ein Zentrierelement für in einen Knochenhohlraum einsetzbare Knochenimplantate, wobei das Zentrierelement vom Knochenimplantat lösbar ausgebildet ist. Die erfindungsgemäß ausgebildeten Hohlkörper-Prothesen umfassen Hilfsmittel, um einen schonenden Operationsverlauf und eine stabile Verankerung der Prothesen im umliegenden Gewebe zu gewährleisten.

## Beschreibung

Die Erfindung betrifft ein Zentrierelement für Endoprothesen sowie ein Knochenimplantat.

Endoprothesen, beispielsweise Femurendoprothesen, werden vielfach mit Hilfe einer Zementierung des Implantats im Knochen verankert, wobei die Knochenzementschicht einerseits das Implantat im Knochen verankert und außerdem zwischen dem Knochen und dem Metallimplantat ähnlich einem Puffer wirkt und auf diese Weise den Knochen als Implantatlager schützt.

Bei der Zementierung ist nachteilig, daß Inhomogenitäten des Knochenzements die theoretisch möglichen vorteilhaften Effekte teilweise aufheben können. Das portionsweise Einbringen von Knochenzement führt immer zu Luft- und Blutbeimischungen, so daß der Knochenzement lamelliert und mechanisch geschwächt wird.

Weiterhin kommt es durch das nachträgliche Einschieben der Endoprothese, insbesondere an Krümmungsstellen des Knochens, zu Abschabungen des Knochenzements vom Knochenlager, so daß an dieser Stelle das Implantat überhaupt nicht vom Knochenzement umschlossen ist. Belastungsspitzen sowohl an dem Implantat wie auch an den Knochenzementgrenzen sind dann zwangsläufig, wobei diese Belastungsspitzen wiederum Brüche des Knochenzements oder auch des Implantats selbst nach sich ziehen können.

Weiterhin entsteht beim Einpressen des Knochenzements ein hoher Überdruck in der Markhöhle, so daß es zu Fettausschwemmungen aus dem Knochenmark kommt, die zu Embolien führen können. Aus diesem Grund wird teilweise der Knochenzement durch Unterdruck in den Knochenhohlraum eingesaugt.

Zu diesem Zweck wird unterhalb des vom Implantat später auszufüllenden Bereichs eine Saugöffnung in den Knochen gebohrt, wobei an dieser Saugöffnung ein Unterdruck angelegt werden kann, der den Knochenzement von der Implantationsstelle des Knochens her in den Knochenhohlraum ansaugt.

Ein Nachteil dieser Methode besteht darin, daß zur Unterdruckerzeugung unterhalb des später eingebrachten Implantats ein Bohrloch im Knochen in dem Abschnitt erforderlich ist, der zur Knochenmitte hin gerichtet ist. Dieses Bohrloch stellt eine mechanische Beeinträchtigung und damit eine Frakturgefährdung für den Knochen dar. Außerdem muß dieser Bohrkanal weitab vom eigentlichen Operationsgebiet angelegt werden, so daß entweder eine Verlängerung der Operationswunde oder eine zusätzliche Operationswunde erforderlich werden.

Der Erfindung liegt die Aufgabe zugrunde, einen dauerhaft festen Sitz des Knochenimplantats sicherzustellen und gleichzeitig einen möglichst schonenden Operationsverlauf zu ermöglichen.

Diese der Erfindung zugrundeliegende Aufgabe wird durch die Ausgestaltung eines Zentrierelements nach Anspruch 1 - 6 sowie eines Knochenimplantats gemäß den Ansprüchen 7 bis 19 gelöst.

Die Erfindung schlägt mit anderen Worten vor, den bekannten Markraumsperrer als Zentrierelement für die Implantatspitze auszubilden, um eine gleichmäßige Ummantelung des Implantats mit Knochenzement zu erreichen.

In einer weiteren Ausbildung kann das Saugloch im Zentrierelement enthalten sein, um die Schwächung des Knochens unterhalb des Implantats zu vermeiden.

Die Erfindung schlägt außerdem vor, ein Zentrierelement mit einem Indikator zu verwenden, wobei der Indikator von außen die Lage für ein anzufertigendes Bohrloch vorgibt, welches durch den Knochen führt. Dieses Bohrloch mündet in ein im Zentrierelement bereits befindliches Bohrloch. Auf diese Weise wird auf Anhieb die richtige Lage des in den Knochen einzubringenden Bohrlochs gewährleistet, so daß ein schonender Operationsverlauf unterstützt wird. Bei diesem Operationsverlauf kann zunächst das Knochenimplantat in den Knochenhohlraum eingeführt werden und anschließend der Knochenzement angesaugt werden, so daß der Hohlraum zwischen dem Implantat und dem Knochen vollständig ausgefüllt wird. Die beim Eintreiben des Knochenimplantats, also der Hohlkörper-Prothese, in den zuvor mit Knochenzement gefüllten Knochenhohlraum, wie sie oben beschrieben sind, können auf diese Weise vermieden werden.

Die Implantation von Fremdkörpern geht mit einer erhöhten Infektionsrate einher, wobei durch die Infektion eine Lockerung des Implantats ausgelöst werden kann und wobei die Infektion an sich eine zum Teil lebensbedrohende Komplikation darstellt.

Die Erfindung löst die ihr zugrundeliegende Aufgabe daher weiterhin durch eine Hohlkörper-Prothese nach Anspruch 3, wobei der Austritt pharmazeutischer Wirkstoffe aus der Prothese dazu beiträgt, das umliegende Gewebe infektionsfrei zu halten, so daß weder eine Komplikation für den Patienten noch eine Lockerung des Implantats auftreten.

Weitere vorteilhafte Ausgestaltungen können den Ansprüchen entnommen werden.

Dabei kann eine Ummantelung vorgesehen sein, die die zusätzlichen Öffnungen der Prothese bei einem in der Prothese anliegenden Unterdruck abdichtet und auf diese Weise die Saugwirkung durch den Hohlraum in der Prothese sicherstellt. Die Ummantelung kann dabei scharfkantig ausgebildete Bereiche der Prothese abdecken und einen mechanischen Schutz für das umliegende Gewebe bieten. Zusätzlich kann die Ummantelung thermisch isolierende oder sogar kühlende Eigenschaften aufweisen, um das umliegende Gewebe vor einer thermischen Schädigung zu schützen, wenn bei der Polymerisation des Knochenzements Temperaturerhöhungen von bis zu 90^{o} auftreten.

Gemäß einem weiteren Merkmal der Erfindung kann die dauerhafte, mechanische Stabilität der Prothesenverankerung dadurch gesichert werden, daß zusätzliche Befestigungsmittel an bzw. in der Prothese vorgesehen sind. Zu diesem Zweck können sich Laschen von der eigentlichen Prothese an die benachbarten Knochenbereiche erstrecken, oder es kann an bzw. in der Prothese eine Aufnahme für Schrauben oder ähnliche Befestigungsmittel vorgesehen sein.

Gemäß einem letzten Gesichtspunkt der Erfindung kann die dauerhafte und sichere Fixierung der Prothese dadurch begünstigt werden, daß die Steifigkeit der Prothese entsprechend beeinflußt wird. Hier ist insbesondere eine Anpassung des Elastizitätsmoduls der Prothese an den Elastizitätsmodul des umgebenden Knochengewebes günstig. Zu diesem Zweck kann der Prothesenhohlraum mit einer dreidimensional gitterartigen Innenstruktur versehen sein oder der Prothesenhohlraum kann mit Stoffen der geeigneten mechanischen Eigenschaften verfüllt werden und so zu einer Verfestigung der Prothese beitragen.

Diese Stoffe können vorteilhaft Pharmaka enthalten und beispielsweise als Antibiotika oder Zytostatika ihre medizinische Wirkung entfalten. Um Belastungsspitzen zu vermeiden und auf diese Weise für eine dauerhaft sichere Fixierung der Prothese zu sorgen, kann die Prothese in bestimmten Fällen auch aus zwei oder mehr Komponenten bestehen, die gelenkig miteinander verbunden sind, so daß die Prothese nur gewisse Kräfte aufnehmen muß, während die Gelenke für eine entsprechende Nachgiebigkeit in anderen Belastungsrichtungen sorgen.

Weitere vorteilhafte Ausgestaltungen der Erfindung sind aus den Ansprüchen zu entnehmen.

Ausführungsbeispiele einer erfindungsgemäßen Hohlkörper-Prothese sind in den Zeichnungen näher beschrieben. Dabei zeigt
- Fig. 1: ein Femurimplantat einer Hüftprothese in perspektivischer und teilweise geschnittener Ansicht,
- Fig. 2: einen Querschnitt durch den Gegenstand von Fig. 1,
- Fig. 3: einen Querschnitt ähnlich Fig. 2 durch ein zweites Ausführungsbeispiel und
- Fig. 4: ein Wirbelsäulenimplantat in perspektivischer Ansicht.

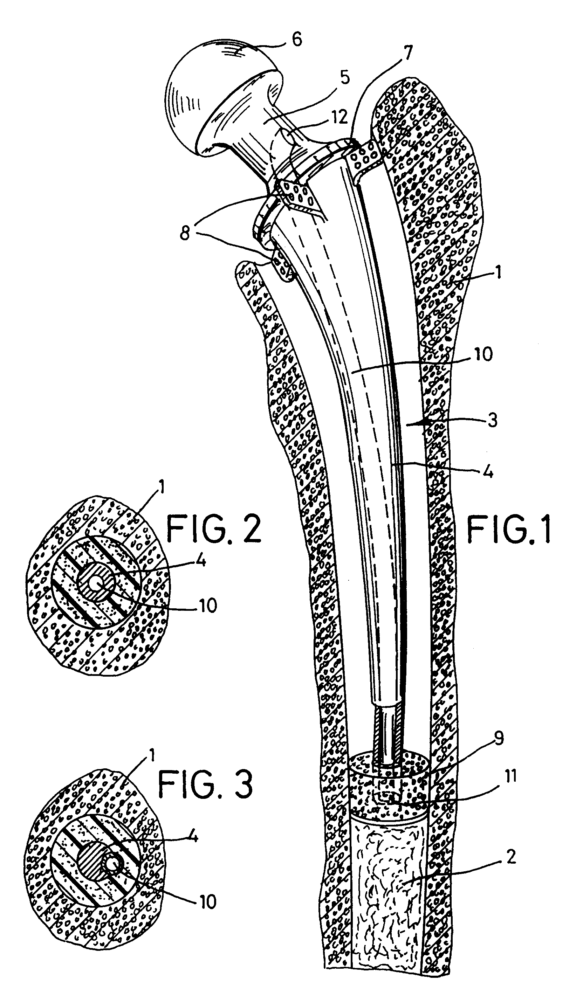

In Fig. 1 ist mit 1 ein Oberschenkelknochen (Femur) bezeichnet, der in seinem Inneren Knochenmark 2 aufweist. Im oberen Bereich des Oberschenkelknochens 1 ist das Knochenmark 2 entfernt worden und mit einem Fräs- oder Raspelwerkzeug ein Hohlraum mit genau definierter Form geschaffen worden.

In diesem Hohlraum befindet sich eine Hohlkörper-Prothese 3, die aus einem Schaft 4, einem Hals 5 und einem Kopf 6 besteht. Im Obergangsbereich zwischen dem Schaft 4 und dem Hals 5 ist ein Kragen 7 angeformt. Zentriervorsprünge 8 fixieren die Hohlkörper-Prothese 3 lagerichtig innerhalb des Oberschenkelknochens 1. Die Zentriervorsprünge 8 sind als einzelne Zentrierfinger unterhalb des Kragens 7 ausgestaltet. In Abwandlung von dem dargestellten Ausführungsbeispiel ist es auch möglich, anstelle der Zentrierfinger den Kragen 7 zu einem Zentrierkragen weiterzubilden, der über Einfüllöffnungen für Knochenzement verfügt. Die Zentriervorsprünge können entweder an der Hohlkörper-Prothese fest angeformt sein oder entfernbar ausgebildet sein, so daß sie beispielsweise zur lagerichtigen Fixierung der Prothese zunächst an der Prothese befestigt werden. Dann wird die Prothese mit den Fixierungen lagerichtig eingesetzt und einzementiert und anschließend werden diese oberen Fixiervorsprünge wieder entfernt.

Im unteren Bereich wird die Prothese 3 durch einen Stopfen 9 zentriert, der porös oder mit Kanälen versehen ist.

Die Hohlkörper-Prothese 3 weist über nahezu ihre gesamte Länge einen durchgehenden Hohlraum 10 in Form einer Durchgangsbohrung auf, wobei die Hohlkörper-Prothese 3 an ihrem untersten Ende innerhalb des Stopfens 9 eine untere Öffnung 11 und im Bereich des Halses 5 eine seitliche, obere Öffnung 12 aufweist, die beide durch den Hohlraum 10 miteinander verbunden sind. Diese Öffnungen stellen Sauganschlüsse dar, mit deren Hilfe innerhalb des Knochens ein Unterdruck angelegt werden kann.

Durch die Zentrierung wird gewährleistet, daß die Hohlkörper-Prothese 3 überall einen nahezu gleichmäßigen Abstand zum umgebenden Oberschenkelknochen 1 aufweist. Dieser, in etwa rohrförmige Hohlraum soll durch Knochenzement ausgefüllt werden, um einen guten und sicheren sowie dauerhaften Sitz der Hohlkörper-Prothese 3 innerhalb des Knochens 1 zu gewährleisten.

Der Knochenzement wird zu diesem Zweck um den Kragen 7 herum zugeführt, während an der oberen Öffnung 12 ein Unterdruck erzeugt, also gesaugt wird. Der Unterdruck breitet sich von dem oberen Sauganschluß (Öffnung 12) durch den Hohlraum 10 und den unteren Sauganschluß (Öffnung 11) sowie durch die Poren oder Kanäle innerhalb des Stopfens 9 aus, so daß anschließend auch im rohrförmigen Hohlraum um die Hohlkörper-Prothese 3 herum ein Unterdruck entsteht. Auf diese Weise wird von oben nachgeführter Knochenzement um die Hohlkörper-Prothese 3 herum in den Knochen 1 eingesaugt.

Wenn der Knochenzement unten am Stopfen 9 angelangt ist, können zwei verschiedene Vorgehensweisen verwendet werden:

Bei einem feinporigen Stopfen 9 kann über den erhöhten Saugwiderstand festgestellt werden, daß der Knochenzement unten am Stopfen 9 anliegt. Aufgrund seiner Viskosität kann der Knochenzement nicht in den Stopfen 9 eindringen. Die Zufuhr weiteren Knochenzementes wird unterbunden. Der Knochenzement kann nun abbinden und gewährleistet nach dem Abbinden den erwünschten sicheren Sitz der Prothese im Knochen.

Der Prothesenhohlraum kann unausgefüllt verbleiben oder mit Material gefüllt werden, welches erwünschte mechanische und/oder pharmakologische Eigenschaften aufweist.

Alternativ dazu kann ein großporiger Stopfen verwendet werden oder ein Stopfen, der in seinem oberen Bereich Ausnehmungen aufweist, die eine Verbindung von dem rohrförmigen Hohlraum um die Hohlkörper-Prothese 3 herum zu dem unteren Sauganschluß (Öffnung 11) schaffen. Bei geeigneter Größe dieser Ausnehmungen oder Poren kann angesaugter Knochenzement durch den Stopfen 9 in die untere Öffnung 11 und damit in den Hohlraum 10 eingesaugt werden, im Hohlraum 10 ansteigen und schließlich an der oberen Öffnung 12 austreten.

Der Austritt des Knochenzementes an der oberen Öffnung 12 stellt dabei einen sicheren Indikator dafür dar, daß der Hohlraum um die Hohlkörper-Prothese 3 herum vollständig mit Knochenzement ausgefüllt ist. Dabei können dem Knochenzement pharmakologische Wirkstoffe zugegeben sein, die Infektionen innerhalb des Hohlraumes 10 und an den Öffnungen 11 und 12 verhindern.

Welche der beiden Befüllungsmethoden angewandt wird, hängt davon ab, ob der Hohlraum 10 mit Knochenzement ausgefüllt werden soll oder nicht. Um einen dauerhaft guten Sitz der Prothese innerhalb des Knochens zu gewährleisten, ist es vorteilhaft, daß die Prothese einen Elastizitätsmodul aufweist, der dem des umgebenden Knochens möglichst ähnlich ist. Durch die Auswahl des Materials für die Hohlkörper-Prothese, für den Zement, und durch die Wahl, ob in der Hohlkörper-Prothese ein Hohlraum 10 verbleibt oder dieser Hohlraum mit Zement verfüllt wird, sind verschiedene Anpassungen und verschiedene Ausgestaltungen der Elastizitätsmodule möglich.

Weiterhin wird durch die Zentrierung der Hohlkörper-Prothese eine gleichmäßig dicke und überall ausreichend dicke Knochenzementschicht um die Hohlkörper-Prothese herum gewährleistet, so daß die Zementschicht nicht an bestimmten, zu dünnen Stellen brechen kann. Ein Bruch der Zementschicht schädigt nicht allein die Zementschicht selbst, sondern führt im längeren Verlauf auch zu Schädigungen der benachbarten Knochenpartien, so daß durch die gleichmäßige Zementschicht in Folge der zentrierten Lage der Hohlkörper-Prothese ein zweifacher Vorteil erzielt wird:

Zunächst bietet die gleichmäßige Ausfüllung des Hohlraumes um die Hohlkörper-Prothese eine optimale mechanische Kraftübertragung von der Hohlkörper-Prothese auf den umgebenden Knochen und zweitens wird der resorbierende Angriff durch Granulationsgewebe auf den Knochen unterbunden, der im Bereich von Stör- oder Bruchstellen des Knochenzementes üblicherweise auftritt.

Der Stopfen 9 kann zusammen mit der Hohlkörper-Prothese 3 in den Knochenhohlraum eingebracht werden. Beim späteren Einbringen der Hohlkörper-Prothese kann die entsprechende Formgebung von Zentrierelement und Prothesenspitze dafür Sorge tragen, daß die Prothese Jagerichtig innerhalb des Knochens fixiert wird.

Soll abweichend von dem dargestellten Ausführungsbeispiel ein Ansaugen von Knochenzement nicht durch die Prothese selbst erfolgen, sondern durch eine seitliche Bohrung, die möglichst tief an bzw. unterhalb der Prothese durch den Knochen verläuft, so kann es vorteilhaft sein, an dem zunächst eingebrachten Zentrierelement einen Indikator anzuordnen, der außerhalb des Knochens die Lage des Zentrierelementes anzeigt. So kann z. B. das Zentrierelement an einem Stab befestigt sein und an diesem Stab in den Knochen eingeführt werden, wobei parallel zu diesem Stab ein zweiter Stab angeordnet ist, der an seiner Spitze außerhalb des Knochens die Lage des Zentrierelementes innerhalb des Knochens anzeigt. Ein derartiger Indikator ist im wesentlichen U-förmig ausgebildet und ermöglicht es, die Saugbohrung durch den Knochen in das Zentrierelement zu richten.

Wenn der Unterdruck am Bereich der oberen Öffnung 12 aufgebaut wird, wirkt dieser Unterdruck über die Kanäle oder Poren des Stopfens 9. Um ein Ansaugen des Knochenmarks 2 zu verhindern, kann der Stopfen 9 an seiner Unterseite abdichtend ausgebildet werden, so daß der Unterdruck nicht über den Stopfen 9 hinaus auf das Knochenmark 2 einwirkt. Auf diese Weise wird verhindert, daß Knochenmark 2 in den Stopfen 9 eingesaugt wird.

Je nach Viskosität des verwendeten Knochenzementes und des Knochenmarks 2 kann diese abdichtende untere Schicht des Stopfens 9 auch entfallen, wenn der Unterdruck lediglich ein Ansaugen des Knochenzements bewirkt, jedoch ein Eindringen des Knochenmarks 2 in den Stopfen 9 nicht zuläßt. Dies hängt vom Zusammenspiel der Viskosität des verwendeten Knochenzementes, der Viskosität des Knochenmarks 2, der Poren- oder Kanalgröße im Stopfen 9 und der Stärke des angelegten Unterdruckes ab.

Fig. 2 zeigt einen Querschnitt durch die Ansicht von Fig. 1 im Bereich des Schaftes 4 der Hohlkörper-Prothese, wobei abweichend zu der Fig. 1 der Bereich zwischen dem Knochen 1 und dem Schaft 4 mit Knochenzement ausgefüllt dargestellt.

Abweichend von dem in Fig. 1 und 2 dargestellten Ausführungsbeispiel kann der Hohlraum auch seitlich an der Prothese angeordnet sein. Er kann einerseits in die Prothese eingeformt sein oder - wie in Fig. 3 angedeutet - in eine Einbuchtung der Prothese eingepaßt sein und als separates Bauteil beispielsweise in Rohrform ausgebildet sein. Dabei kann das Rohr als entfernbarer Hohlkörper nach der Operation an der Prothese verbleiben und hohl bleiben oder gefüllt werden oder er kann nach dem Einbringen der Prothese und des Knochenzementes entfernt werden.

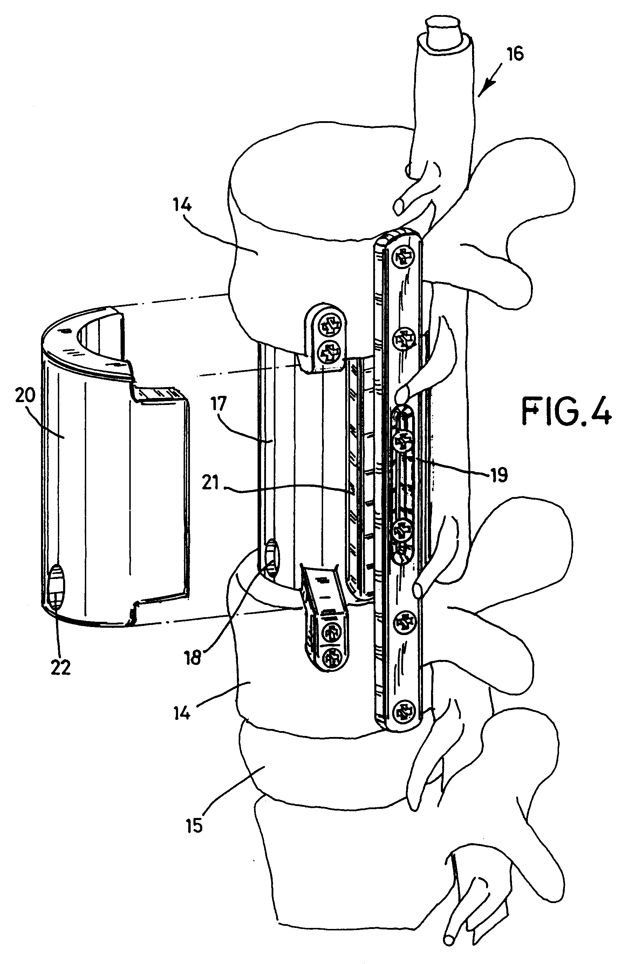

Fig. 4 zeigt eine Wirbelsäulenprothese, wobei schematisch eine Wirbelsäule mit mehreren Wirbelknochen 14, Bandscheiben 15 und einem Rückenmark 16 dargestellt ist. Eine rohrförmige Hohlkörper-Prothese 17 ist zwischen zwei Wirbelknochen 14 eingesetzt und weist ebenfalls zwei Öffnungen zum Einsaugen des Knochenzementes auf, wobei von den beiden lediglich eine Öffnung 18 dargestellt ist.

Neben der Hohlkörper-Prothese 17 sind Fixierungsplatten 19 vorgesehen, die durch Schrauben an ihren beiden Enden die beiden durch die Hohlkörper-Prothese 17 verbundenen Wirbelknochen 14 gegen Verdrehungen festlegen. Weiterhin weisen die Fixierungsplatten 19 in ihrem mittleren Bereich Langlöcher auf, durch die sich Schrauben in die Hohlkörper-Prothese 17 zu deren achsengerechter Fixierung erstrecken. Die Hohlkörper-Prothese 17 kann zu diesem Zweck vorbereitete Aufnahmen für die Schrauben aufweisen.

Alternativ hierzu oder zusätzlich sind an der Hohlkörper-Prothese 17 Laschen vorgesehen, die sich in den Bereich des benachbarten gesunden Knochengewebes erstrecken und eine Fixierung der Hohlkörper-Prothese 17 direkt an den benachbarten Wirbelknochen 14 ermöglichen.

Beim Abbinden des Knochenzements treten unerwünscht hohe Temperaturen auf, die das empfindliche Rückenmark 16 möglicherweise schädigen könnten. Es ist daher bei derartigen Wirbelsäulenoperationen notwendig, das Rückenmark 16 gegen die Hitzeentwicklung des abbindenden Knochenzementes zu schützen. Zu diesem Zweck ist ein zweiteiliger Mantel schematisch dargestellt, der aus zwei Mantelhälften 20 und 21 besteht. Die Mantelhälften weisen Öffnungen 22 auf, durch welche die Einlaß- bzw. die Auslaßöffnung 18 der Hohlkörper-Prothese erreichbar sind, so daß die Hohlkörper-Prothese 17 bei angelegtem Mantel mit Knochenzement gefüllt werden kann. Der Mantel kann dabei isolierende Eigenschaften aufweisen oder ein Kühlmittel enthalten oder Kanäle für den Durchfluß eines Kühlmittels enthalten.

Der Mantel ist aus den Mantelhälften 20 und 21 aufgebaut, damit er nach der Abkühlung des Knochenzementes entfernt werden kann. Die abgeschlossene Wandung der Hohlprothese 17 und die Entfernbarkeit des Mantels stellen sicher, daß nach Einbringen der Hohlkörper-Prothese 17 eine definierte Ausdehnung des Knochenzementes sichergestellt ist, so daß weder der Knochenzement noch - durch die Entfernbarkeit - der Mantel das Rückenmark schädigen kann. Ein entsprechend weicher Mantel kann ggf. auch an der Prothese verbleiben, ohne daß nahe Gewebebereiche geschädigt werden.

Auch in der Hohlkörper-Prothese 17 kann der eingebrachte Knochenzement pharmakologische Wirkstoffe enthalten, die als Antibiotikum oder Zytostatikum wirken. Dabei kann die Hohlkörper-Prothese 17 Öffnungen aufweisen, die den Durchtritt dieser Wirkstoffe durch die Prothesenwandung zum Gewebe ermöglichen. Die Hohlkörper-Prothese 17 kann dazu z. B. gitter- oder netzartig ausgebildet sein.

Die Ummantelung trägt in diesem Fall dafür Sorge, daß zunächst nur die Sauganschlüsse frei sind. Nach Einsaugen des Knochenzements und ggf. nach dessen Abkühlung wird die Ummantelung entfernt, so daß die Pharmaka auf das umliegende Gewebe einwirken können.

## Patentansprüche

1. Zentrierelement für in einen Knochenhohlraum einsetzbare Knochenimplantate, dadurch gekennzeichnet, daß das Zentrierelement (9) am Knochenimplantat in dessen Endbereich lösbar ausgebildet ist.

2. Zentrierelement nach Anspruch 1, dadurch gekennzeichnet, daß das Zentrierelement (9) im Bereich der Implantatspitze ausgebildet ist.

3. Zentrierelement nach Anspruch 1, dadurch gekennzeichnet, daß das Zentrierelement (8) im Bereich der Implantationsstelle des Knochens (1) ausgebildet.

4. Zentrierelement nach Anspruch 2 und/oder 3, dadurch gekennzeichnet, daß das Zentrierelement ein oder mehrere Vorsprünge aufweist.

5. Zentrierelement nach einem oder mehreren der vorangehenden Ansprüche, dadurch gekennzeichnet, daß das Zentrierelement als Zentrierkragen ausgebildet ist.

6. Zentrierelement nach einem oder mehreren der vorangehenden Ansprüche, dadurch gekennzeichnet, daß das Zentrierelement innen und/oder außen Kanäle aufweist.

7. Zentrierelement nach einem oder mehreren der vorangehenden Ansprüche, gekennzeichnet durch eine zumindest teilweise mikroporöse Struktur.

8. Zentrierelement nach einem oder mehreren der vorangehenden Ansprüche, gekennzeichnet durch eine zumindest teilweise makroporöse Struktur.

9. Zentrierelement nach einem der Ansprüche 7 und/oder 8, dadurch gekennzeichnet, daß der implantatferne Bereich des Zentrierelementes undurchläßig und der implantatnahe Bereich des Zentrierlementes durchlässig ausgebildet ist.

10. Zentrierelement nach einem oder mehreren der Ansprüche 7 bis 9, gekennzeichnet durch einen gasdichten Abschluß des Zentrierelementes (9) zur Markhöhle.

11. Zentrierelement nach einem oder mehreren der vorangehenden Ansprüche, dadurch gekennzeichnet, daß das Zentrierelement eine seitliche Öffnung aufweist.

12. Zentrierelement nach Anspruch 11, dadurch gekennzeichnet, daß die seitliche Öffnung in einen Kanal mündet.

13. Zentrierelement nach einem oder mehreren der vorangehenden Ansprüche, dadurch gekennzeichnet, daß das Zentrierelement eine zum Implantat hin gerichtete Öffnung aufweist.

14. Zentrierelement nach Anspruch 13, dadurch gekennzeichnet, daß die Öffnung in einen Kanal mündet.

15. Zentrierelement nach einem oder mehreren der vorangehenden Ansprüche, gekennzeichnet durch einen Indikator, der die Lage des im Knochen befindlichen Zentrierlementes außen anzeigt.
